# EUROPEAN PATENT SPECIFICATION

(11) **EP 0 533 297 B1**
(45) Date of publication and mention of the grant of the patent: **19.11.1997**
(21) Application number: 92203126.5
(22) Date of filing: 10.03.1986
(51) Int. Cl.: A61K 9/16, A61K 9/22, A61K 31/18

(54) **Controlled-release pharmaceutical formulations**
Pharmazeutische Formulierung mit gesteuerter Freigabe
Formulation pharmaceutique à libération contrôlée

(30) Priority: 08.03.1985 JP 46180/85
(43) Date of publication of application: 24.03.1993
(62) Divisional of application: 86301700.0
(73) Proprietor: YAMANOUCHI PHARMACEUTICAL CO., LTD., Tokyo 103 (JP)
(72) Inventor: Fukui, Muneo, Urawa-shi, Saitama (JP); Tomuro, Kouji, Shizuoka-shi, Shizuoka (JP); Masuyama, Shigeru, 1-8 Azusawa 1-chome Itabashi-ku Tokyo (JP); Kajiyama, Atushi, 1-8 Azusawa 1-chome Itabashi-ku Tokyo (JP); Hikosaka, Tamio, 1-8 Azusawa 1-chome Itabashi-ku Tokyo (JP); Aruga, Masayoshi, Ageo-shi, Saitama (JP); Soeishi, Yoshiaki, 1-8 Azusawa 1-chome Itabashi-ku Tokyo (JP); Higuchi, Saburo, Hasuda-shi, Saitama (JP)
(74) Representative: Geering, Keith Edwin

(56) References cited:
- EP-A- 0 034 432
- EP-A- 0 187 703
- DD-A- 104 027
- DE-A- 1 467 781
- DE-A- 1 962 016
- DE-A- 2 148 391
- DE-A- 2 921 931
- GB-A- 630 439
- GB-A- 2 075 839
- US-A- 4 489 026
- PATENT ABSTRACTS OF JAPAN, unexamined applications, C field, vol. 9, no. 160, 4th July 1985, THE PATENT OFFICE JAPANESE GOVERNMENT, page 144 C 289; & JP-A-60 036 410 (UNITIKA K.K.) 25-02-1985

## Description

This invention relates to a novel and effective pharmaceutical controlled-release formulation for oral administration.

When a controlled-release pharmaceutical formulation is administered to a living body, intra-or inter-individual variations in effect occur frequently influenced by factors in the pharmaceutical formulation or factors in the living body. One of the factors in the living body is a variation in gastrointestinal transit time. A formulation which reduces the effect of this factor is known (H.Bechgaad and G.H.Nielsen, Drug Devel.Ind. Pharm., 4, 53[1978]. This is a formulation form (such as tablets, hard capsules, etc.) which disintegrates in the gastrointestinal tract to form a number of particles (e.g., microcapsules, microspheres, etc.,) which are distributed broadly in the gastrointestinal tract and from which an active substance is gradually released.

Hitherto, there are known various materials and various production processes for obtaining individual particles (e.g.,microcapsules, microspheres etc.,) of controlled-release pharmaceutical formulations containing an active substance.

For example, waxes, lipids, water-insoluble macromolecular materials, ion-exchange resins, etc., are known as the above-mentioned materials. A production process for individual particles frequently requires a complicated and long step of preparing granules containing an active substance and other material(s) and applying thereto an enteric coating. In such a production process the production cost is frequently high and the reproducibility of the dissolving characteristics of products is not good.

As a material for forming a structured particle that is not easily disintegrated in the gastrointestinal tract, crystalline cellulose (formerly "microcrystalline cellulose") is known. A pharmaceutical formulation using crystalline cellulose in an amount of about 10 to 40% by weight based on the weight of the formulation is described in Japanese Patent Publication No. 5275/70. The above-described patent described a controlled-release pharmaceutical formulation containing as active substances bis (o-benzoyl-thiamine)disulfide, but an enteric coating is necessary in order to prolong the release time The pharmaceutical formulation of the patent has a structure which does not easily disintegrate in the gastrointestinal tract. It is known than if the amount of crystalline cellulose is about 10 to 40% by weight, the pharmaceutical formulation is insufficiently strong and also generally provides insufficient controlled-release of active substance.

Furthermore, European Patent Publication 80341A describes an invention of "oral pharmaceutical controlled release multiple-units formulation". However, in the invention, "cores" are produced by a complicated process and also enteric coating is applied thereto for obtaining controlled-release.

Moreover, the above-described pharmaceutical formulation does not disintegrate in the stomach and is prepared with the addition of disintegrants so that the coating is eroded and the core itself disintegrates in the small intestine.

DD-A-104027 discloses pharmaceutical compositions comprising an active, carrier (chitin, cellulose etc.), and binder (ethylcellulose etc.). GB-A-2075839 discloses sustained release granular pharmaceutical preparations comprising an active, binding agent (such as ethyl cellulose, copolymers of methacrylic acid and methyl methacrylate), and C₁₆ - C₁₈ saturated fatty acid or metal salt thereof; microcrystalline wax may be present as a diluent. DE-A-1467781 discloses pharmaceutical compositions comprising 100 g of an active, 140 g carboxymethylcellulose and 30 g polyvinylacetate. EP-A-0187703 discloses sustained release granular pharmaceutical tablets comprising an active (12.5 parts), a carrier (87 parts of chitosan), and 12.5 parts of magnesium stearate. GB-A-630439 discloses prolonged continuous release pharmaceutical compositions comprising 2 g of ephedrine sulphate, 20 g of gypsum plaster and 17 cm³ of water. DE-A-2148391 discloses prolonged release pharmaceutical compositions comprising 100 g of lactose, 15 g of ethisteron and 15 g of cellulose-acetate-phthalate.

The present invention provides a controlled-release pharmaceutical formulation suitable for oral administration which shows a reproducible dissolution rate without enteric coating and which can be simply produced.

Thus, the invention provides a pharmaceutical controlled-release formulation which is a granulation product characterised in that the granules do not disintegrate but gradually releases the physiologically active substance in the gastrointestinal tract and in that they are obtainable by a method comprising adding [i] a macromolecular water-insoluble release controlling agent selected from acrylic acid polymers and acrylic acid copolymers to a mixture of [ii] physiologically active substance and [iii] crystalline cellulose as carrier substance and granulating the resulting mixture, said crystalline cellulose constituting at least 50% of the weight of the formulation, the release control agent being in the form of an aqueous suspension or emulsion or aqueous organic solvent solution.

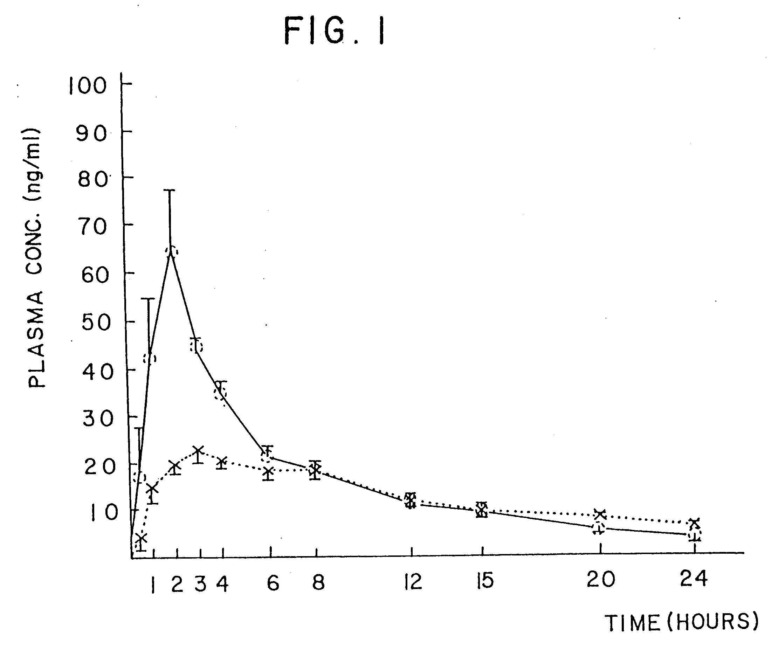

Fig. 1 is a graph showing the change with the passage of time of the concentration of physiologically active substance 5-{2-[2-(o-ethoxyphenoxy)ethylamino]propyl}-2-methoxybenzenesulfonamide hydrochloride (hereinafter referred to as YM-12617) in plasma after orally administering the controlled-release pharmaceutical formulation of this invention as tablets to humans.

The above-described granulation product (active substance-containing granules) for use in this invention may be water-permeable but is not substantially disintegrated - (i.e., scarcely disintegrates or does not disintegrate for at least a few hours) in the gastro-intenstinal tract. Also, the pharmaceutical formulation of this invention has a high physical strength and the product is rarely crushed when formed into tablets by compression. Furthermore, by properly selecting an enteric coating agent and properly controlling the compounding ratio thereof in the preparation of the granulation product (active substance-containing granules), a granulation product having the desired dissolving characteristics can be obtained.

The purpose of the carrier is to provide, on granulation, cohesive units (granules) containing the active substance. The material used as carrier is capable of forming a structured particle of the formulation.

The material used as the carrier in this invention is crystalline cellulose. The amount of carrier is at least 50% by weight based on the weight of the granules.

The release controlling agent in this invention is involved as a binding agent for granulation. The release controlling agents are used in the form of an aqueous suspension, an aqueous emulsion, or a water-containing organic solvent solution. The acrylic polymers are also commercially available as, for example, Eudragit L 30 D (Röhm Co., trade name; aqueous suspension of methacrylic acid-ethyl acrylate copolymer), Eudragit E 30 D (aqueous suspension of an ethyl acrylate-methyl methacrylate copolymer), etc. They can be used as the release controlling agent as they are or diluted with water.

There is no particular restriction on the amount of the release controlling agent but the amount suitable for wet granulation may be used. There is also no particular restriction on the concentration of the release controlling agent (as an aqueous liquid material) but since if the compounding ratio of the water-insoluble polymer is high, the release of the physiologically active substance is delayed, the amount of the release controlling agent (as aqueous liquid material) used may be suitably selected. Although there is no particular restriction on the amount of the release controlling agent, generally the amount is 0-30% (as solid component) or 50-150% (as aqueous liquid material) by weight based on the weight of the granules.

In this invention, for controlling the dissolving characteristics of an active substance, an alkaline earth metal salt ( or an alkaline metal salt) of a higher fatty acid or an enterosoluble polymer may be added to the formulation.

The addition of the aforesaid material is effective when the physiologically active substance is a so-called micro-medicament. Examples of the alkaline earth metal salt or alkaline metal salt of a higher fatty acid are magnesium stearate, and calcium stearate. Also, examples of the enterosoluble polymers are cellulose acetate phthalate, hydroxypropylmethyl cellulose phthalate, and a methacryclic acid-methyl methacrylate copolymer (Eudragit L, S). The compounding amount thereof is usually 1 to 15% by weight.

When such enterosoluble polymer is used a plasticiser such as PEG 6000, Tween 80 (trade name), and triacetin, may be added.

The compounding amount thereof is usually 0 to 15% by weight based on the weight of the release controlling agent (solid component). In addition, an alkaline metal halide or an alkaline earth metal halide, such as sodium chloride or calcium chloride can be used for the same purpose.

As decribed above, the release of the physiologically active substance can be controlled by selecting the kind of the release controlling agent and/or controlling the compounding amount of the alkaline earth metal salt (or alkaline metal salt) of a higher fatty acid or an enterosoluble polymer. According to the characteristics of the active substance, the release thereof can be also delayed by subjecting the active substance itself to a hydrophobic treatment. The hydrophobic treatment can be performed by micro-capsulating the active substance by, for example a spray congealing method used wax. Examples of the wax which may be used for the purpose are hydrogenated vegetable oils such as hydrogenated castor oil.

There is no particular restriction on the physiologically active substance for use in this invention. The amount of the active substance is generally less than 30% by weight based on the weight of the granules.

In the test examples and examples described hereinbelow, 5-{2-[2-(o-ethoxyphenoxy)ethylamino]propyl]-2-methoxybenzenesulfonamide hydrochloride (YM-12617) having a relatively low solubility in water (about 0.3 to 0.5%) was used as the active substance. Active substances having a high solubility can also, as a matter of course, be used in this invention.

YM-12617 shows an α-blocking action and can be used for the treatments of hyperpiesia, cardiac insufficiency, lower urinary desease, etc.

The formulation of this invention may also contain any of the usual additives or excipients such as filler, and coloring agent.

The mixture of physiologically active substance, carrier substance and optionally alkaline earth metal salt (or an alkaline metal salt) of a higher fatty acid or an entersoluble polymer is granulated after the addition of the above-described aqueous liquid material as a release controlling agent. The granulation is performed by an agitation-type apparatus, a rotation-type apparatus, a centrifugal-type apparatus, a fluidized bed-type apparatus, or an apparatus of mixed types.

The size (diameter) of the granulation product - (particles) is 0.1 to 1.5 mm, preferably 0.2 to 1.0 mm.

The individual active-substance-containing particles thus obtained are formed into larger units such as tablets, capsules, granules etc.

The active substance-containing particles of this invention have a high mechanical strength, and mostly do not disintegrate when formed into tablets.

They disperse widely in the gastrointestinal tract when they are administered to a living body. Also, they are water-permeable but do not substantially disintegrate; they gradually release active substance into the gastrointestinal tract, whereby a long controlled release can be attained. Also, intra-and inter-subject variations are very small and the physiological effects are excellent in reproducibility. Furthermore, the pharmaceutical formulation of this invention can be obtained by a simple and safe production process.

Tests and results showing dissolution characteristics and concentrations in plasma of the active substance of the controlled-release formulation of this invention are described below.

### (1) Dissolution Test:

### Test Procedure:

The test was performed by the paddle method of the 2nd dissolution test method in the Japan Pharmacopoeia. That is, the test was performed by a UV method or a liquid chromatograph method (shown below) at a rotation speed of the paddle of 150 r.p.m., using 500 ml of a 1st liquid (artificial gastric juice) of the Japanese Pharmacopoeia and 500 ml of a 2nd liquid (artificial intestinal juice) of the Japanese Pharmacopoeia, respectively. A sample was first tested in the 1st liquid for one hour followed by the test in the 2nd liquid for one hour.

### (i) UV Method

The pharmaceutical formulation obtained in each example shown below was used as a sample. The amount of each sample corresponding to 50 mg of YM-12617 was subjected to the aforesaid dissolution test, the dissolved liquid was filtered, and the active substance in the filtrate was determined at a detection wavelength of 278 nm.

### (ii) High Performance Liquid Chromatograph Method (HPLC Method):

The pharmaceutical formulation prepared in each example was used as a sample. The amount of the sample corresponding to 1 mg of YM-12617 was subjected to the aforesaid dissolution test, the dissolved liquid was filtered, and the active substance was determined under the following conditions.

### Operation Conditions:

Detector: Ultraviolet Spectrophotometer (Detection wavelength of 225 nm)
Column: In a stainless tube of about 4 mm inside diameter and about 150 mm length was packed about 5 µm of octadecylsililated silica gel (e.g., Nucleosil 5C18, trade name) as a packing agent
Column Temperature: About 35°C.
Mobile Phase: Mixture of 0.05N perchloric acid and acetonitrile (7 : 3).
Flow rate: Constant amount of 0.8 to 1.5 ml per minute.

### Test Results:

The results thus obtained are shown in Table 1.

**Table 1**

| Example No. | Sample % of YM-12617 in the units (w/w) | Dissolution Rate(%) | | | Note |
|---|---|---|---|---|---|
| | | Test Method | 1st Liquid* (1 hour) | 2nd Liquid* (1 hour) | |
| 1 | 1 | UV | 49.6 | | (A)** |
| 12 | " | HPLC | 50.3 | 57.6 | " |
| 4 | " | UV | 45.6 | | " |
| 5 | 0.5 | UV | 52.4 | 66.2 | " |
| 13 | " | HPLC | 60.4 | 72.6 | " |
| 6 | " | UV | 54.6 | | " |
| 8 | 1 | UV | 42.7 | | (B)** |
| 9 | " | " | 29.2 | | " |
| 10 | " | " | 32.5 | | " |
| 11 | 0.5 | " | 30.9 | | " |

| | | | | | |
|---|---|---|---|---|---|
| (*): By the Japan Pharmacopoeia | | | | | |
| (**): (A): Eudragit L30D-55 was used as the release controlling agent (B): Particles containing magnesium stearate, The release controlling agent was same as (A). | | | | | |

### (2) Absorption Studies following Oral Administration:

### (A)

(i) Tablets obtained in Example 12 were used as the sample of this invention and conventional tablets obtained in Reference Example 1 were used as a control. A dose corresponding to 1 mg of YM-12617 was orally administered to five adult male subjects, respectively, by a cross over method. Then, blood samples were withdrawn at definite time intervals and the concentration of the active substance in plasma was measured by the method shown below.
(ii) Determination Method of YM-12617 in Plasma: After adding 0.5 ml of an aqueous solution of internal standard substance (containing 0.5µg of amosulalol hydrochloride) to 1.5 ml of plasm, 1 ml of a saturated aqueous solution of sodium hydrogencarbonate was added thereto and the active substance was extracted with 4 ml of ehtyl acetate. The ethyl acetate extract was further extracted with 2.5 ml of 0.4N hydrochloric acid. The hydrochloric acid layer thus obtained was adjusted to weak alkaline by the addition of 2 ml of a saturated aqueous solution of sodium hydrogencarbonate and then re-extracted with 4 ml of ethyl acetate. After adding 0.05 ml of an aqueous solution of 0.1M sodium hydrogencarbonate and 0.1 ml of an acetone solution of 500 µg of dansyl choride, the ethyl acetate layer thus obtained was distilled under reduced pressure for 120 minutes at 35°C. After adding 4 ml of ether to the mixture, the organic layer thus formed was washed with 5 ml of water and then with 5 ml of an aqueous solution of 0.2 N hydrochloric acid. The solvent was distilled off from the organic layer, the residue thus formed was dissolved in 0.05 ml of a mixture of benzene and methanol (100 : 1), and using all of the solution the active substances was determined by a liquid chromatography under the following operation condition. The retention times for dansyl-YM-12617 and dansyl-amosulalol when the flow rate of the eluent was 1.4 ml/min. were 8.1 minutes and 12.5 minutes, respectively.

### Operation Condition

Detector: Fluorescent Photometer (Excitation wavelength 365 n m, fluorescent wavelength 500 n m )
Column: In a stainless tube of about 4 mm in inside diamater and about 250 mm in length was packed by about 5 µm of silica gel (e.g.,Lichrosorb Sl 100, trade name, made by Merck & Co., Ltd.) as a filler.
Column Temperature: About 10°C
Flow Rate: Constant flow rate of 1.2 to 1.9 ml per minute.

(iii) The results thus obtained are shown in Table 2, Table 3, and Fig. 1.
   In Fig. 1, 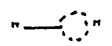 shows tablets obtained in Reference Example 1, and "-----X" shows tablets obtained in Example 12.

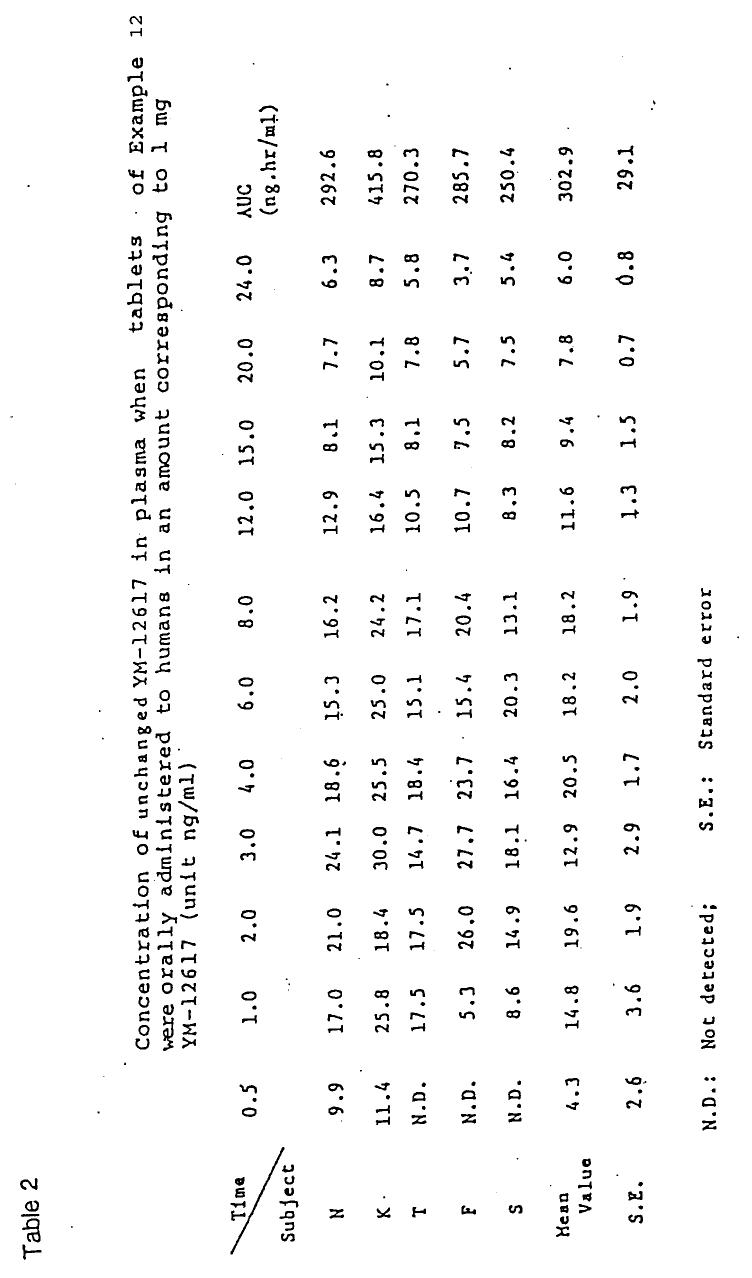

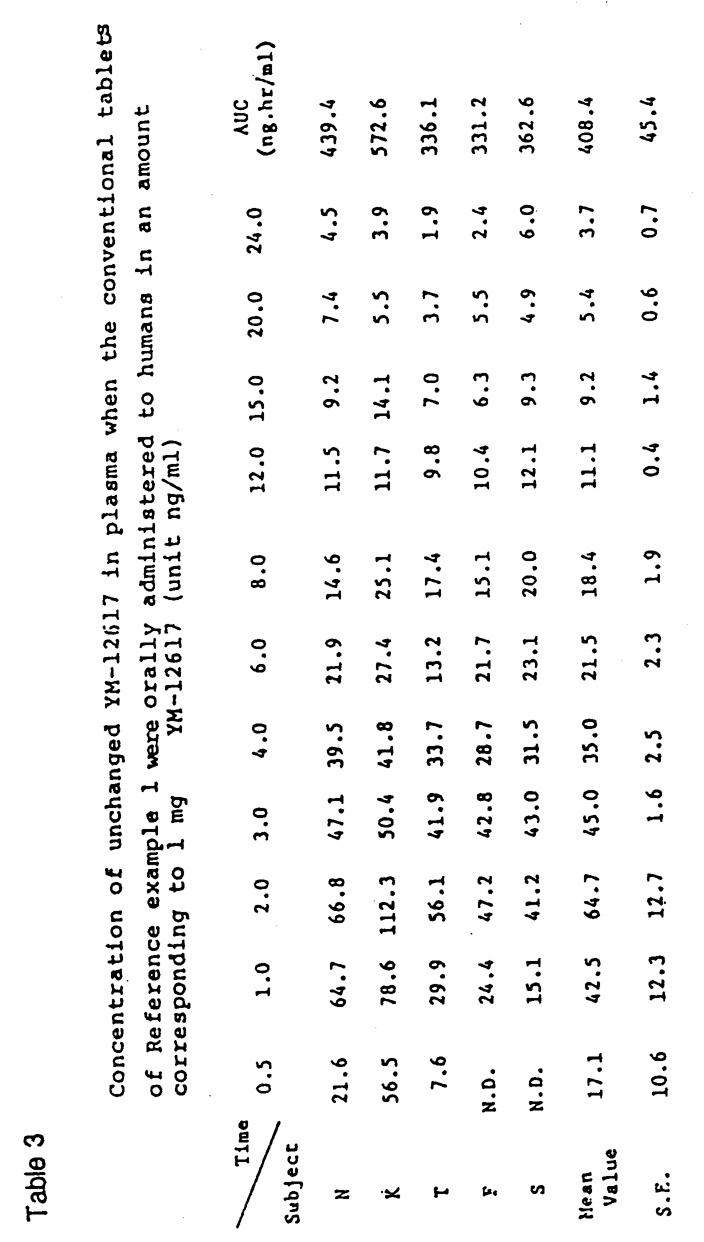

As is clear from Fig. 1, in the case of administering the tablets prepared in Example 12 the concentration pattern of the active susbtance in plasma was good and showed the following features.
a) The ratio of Cmax/Cmin is small, which shows long acting characteristics.
b) Intra-individual variation is small.

### (3) Relationship between dissolution characteristics and rotation speed in dissolution tests.

The rotation speed of the paddle in the dissolution test (1) described above was changed and the influence of the rotation speed on the dissolution rate was studied. (The active substance was determined by UV method.). The results thus obtained are shown in Table 4.

**Table 4**

| Example No. | % dissolved, JP, 1st Liquid*, After 1 Hour. | | | |
|---|---|---|---|---|
| | Rotation speed of the Paddle | | | |
| | 50 rpm | 100 rpm | 150 rpm | 200 rpm |
| 1 | 42.1 | 45.7 | 45.4 | 45.3 |

| | | | | |
|---|---|---|---|---|
| (*): The 1st liquid in Tables 4 and 6 above is artificial gastric juice in the Japan Pharmacopoeia. | | | | |

As is clear from Table 4, it can be seen that there is no change of the dissolution characteristics with change of rotation speed. Hence, the motion of the gastrointestinal tract in the living body scarcely influences the dissolution rate of the formulation of this invention.

### (4) Stability of Dissolution Characteristics with the Passage of Time:

The product obtained in each example was stored under severe conditions (shown in Table 5 below) for one month and then the dissolution test as in above-described test (1) was performed on the product. In this case, the determination of the active substance was performed by UV method.

The results thus obtained are shown in Table 5.

**Table 5**

| Test No. (Example No.) | Dissolution Rate (%) | | |
|---|---|---|---|
| | Initial (%) | Value After 1 Month | |
| | | 50°C, Closed | 40°C, 75% RH |
| 4 | 45.6 | 45.5 | - |
| 11 | 32.9 | 37.1 | 34.2 |

As is clear from the above results when the samples of this invention are stored under the severe conditions, the change in the dissolution characteristics is very small and thus, the formulations are stable to the passage of time.

### (5) Good Dissolution Reproducibility:

Three samples were prepared by the same manner as in Example 4 and the dissolution test as above was performed on each sample (the determination of the active substance was performed by UV method). The results thus obtained are shown in Table 6.

**Table 6**

| Sample No. (Example. No.) | % dissolved JP, 1st Liquid* (1 hour) |
|---|---|
| Example 4 | 45.6 |
| 4 - 1 | 45.4 |
| 4 - 2 | 45.9 |
| 4 - 3 | 45.3 |

| | |
|---|---|
| (*): The 1st liquid in Tables 4 and 6 above is artificial gastric juice in the Japan Pharmacopoeia. | |

From the results shown in Table 6 it can be seen that the samples of this invention show good dissolution reproducibility.

Examples of the invention will now be described below, followed by Reference Examples 1 and 2.

### Example 1 (Production of active substance-containing granules)

After sufficiently mixing 5 g of YM-12617 and 470 g of crystalline cellulose, a mixture of 83.3 g (25 g as solid component) of Eudragit L30D-55(Röhm Co.) and 500 g of water was added to the aforesaid mixture and the resultant mixture was granulated by a high-speed mixer. The granules obtained were spheres having particle sizes of 0.1 to 1.5 mm, mainly 0.2 to 1.0 mm.

### Examples 2 to 7

By following the same procedure as Example 1 using the formulae shown in Table 7 below, active substance-containing granules were prepared.

**Table 7**

| Formula (g) | Example No. | | | | | |
|---|---|---|---|---|---|---|
| | 2 | 3 | 4 | 5 | 6* | 7 |
| YM-12617 | 5 | 5 | 5 | 2.5 | 2.5 | 1.25 |
| Crystalline Cellulose | 445 | 395 | 482.5 | 472.5 | 472.5 | 473.75 |
| Eudragit L30D-55 (Solid comp.) | 166.6 (50) | 333.3 (100) | 41.7 (12.5) | 83.3 (25) | 83.3 (25) | 83.3 (25) |

| | | | | | | |
|---|---|---|---|---|---|---|
| (*): Centrifugal fluidized bed granulator was used. | | | | | | |

### Example 8

After sufficiently mixing 5 g of YM-12617, 420 g of crystalline cellulose, and 50 g of magnesium stearate, a mixture of 83.3 g (25 g as solid component) of Eudragit L30D-55 and 500 g of water was added to the aforesaid mixture and the resultant mixture was kneaded and granulated by a centrifugal fluidized bed granulator. The granules obtained were spheres having particle sizes of 0.1 to 1.5 mm, mainly 0.2 to 1.0 mm.

### Examples 9 to 11

By following the same procedure as Example 8 using the formulae shown in Table 8 active substance-containing granules were prepared.

**Table 8**

| Formula (g) | Example No | | |
|---|---|---|---|
| | 9 | 10 | 11 |
| YM-12617 | 5 | 5 | 2.5 |
| Crystalline Cellulose | 460 | 445 | 462.5 |
| Magnesium Stearate | 10 | 25 | 10 |
| Eudragit L30D-55 (Solid component) | 83.3 (25) | 83.3 (25) | 83.3 (25) |

### Example 12

To 20 g of the particles (active substance-containing granules obtained in Example 1 were added 44.9 g of lactose, 20 g of starch, 9.7 g of crystalline cellulose, 5 g of CMC-Ca, and 0.5 g of magnesium stearate and tablets were prepared using the mixture thus obtained by a conventional method (one tablet of 100.1 mg contained 0.2. mg of YM-12617).

### Example 13

After sufficiently mixing 40 g of the granules obtained in Example 5, 24 g of lactose, 34.54 g of crystalline cellulose, 12 g of low-substituted hydroxypropyl cellulose, and 3 g of corn starch, 40 g of 10% corn starch paste was added to the mixture and the resultant mixture was granulated by a conventional method. Then, 2.4 g of a hydrogenated oil and 0.06 g of calcium stearate were added to the granules thus obtained and tablets were manufactured using the resultant mixture by a conventional method (one tablet of 120 mg contained 0.2 mg of YM-12617).

### Example 14

After sufficiently mixing 5 g of YM-12617 and 467.5 g of crystalline cellulose, a mixture obtained by adding 414.2 g of water and 2.5 g of PEG 6000 to 83.3 g (25 g as solid component ) of Eudragit L30D-55 (trade name) was added to the aforesaid mixture and the resultant mixture was granulated by a high-speed mixer. The granules were spheres having particle sizes of 0.1 to 1.5 mm, mainly 0.2 to 1.0 mm.

### Reference Examples 1 and 2

Conventional tablets were prepared using the formulae shown in Table 9 below.

**Table 9**

| Formula | Reference Example No. | |
|---|---|---|
| | 1 | 2* |
| YM-12617 | 0.2 g | 2.5 g |
| Lactose | 66.7 g | 63.0 g |
| Starch | 28.6 g | - |
| Starch (for paste) | 3.5 g | - |
| Magnesium Stearate | 1.0 g | 1.0 g |
| Corn Starch | - | 30.0 g |
| Corn Starch (for paste) | - | 3.5 g |
| tablet | 100 mg | 100 mg |

| | | |
|---|---|---|
| (*): Prepared by fluidized bed granulator. | | |

## Claims

1. A pharmaceutical controlled-release formulation which is a granulation product characterised in that the granules do not disintegrate but gradually release the physiologically active substance in the gastrointestinal tract and in that they are obtainable by a method comprising adding [i] a macromolecular water-insoluble release controlling agent selected from acrylic acid polymers and acrylic acid copolymers to a mixture of [ii] physiologically active substance and [iii] crystalline cellulose as carrier substance and granulating the resulting mixture, said crystalline cellulose constituting at least 50% of the weight of the formulation, the release control agent being in the form of an aqueous suspension or emulsion or aqueous organic solvent solution.

2. A pharmaceutical formulation as claimed in claim 1 wherein the release controlling agent [i] is a methacrylic acid-ethyl acrylate copolymer or a mixture of said copolymer and ethyl cellulose.

3. A pharmaceutical formulation as claimed in any preceding claim wherein the physiologically active substance [ii] is 5-{2-[2(o-ethoxyphenoxy)ethylamino]propyl}-2-methoxy-benzenesulfonamide hydrochloride (YM-12617).

4. A pharmaceutical formulation as claimed in any preceding claim wherein the product granules have diameters of 0.1 to 1.5 mm.

5. A granular formulation according to any preceding claim in tablet or capsule form.

## Patentansprüche

1. Pharmazeutische Formulierung zur gesteuerten Freisetzung, die ein Granulationsprodukt ist, dadurch gekennzeichnet, daß die Körner nicht zerfallen, sondern nach und nach die physiologisch aktive Substanz in dem Gastrointestinaltrakt freisetzen und daß sie erhältlich sind nach einem Verfahren, umfassend die Zugabe [i] eines makromolekularen wasserunlöslichen, die Freisetzung steuernden Mittels, ausgewählt aus Acrylsäurepolymeren und Acrylsäurecopolymeren, zu einem Gemisch aus [ii] physiologisch aktiver Substanz und [iii] kristalliner Cellulose als Trägersubstanz und Granulieren des erhaltenen Gemisches, wobei die kristalline Cellulose mindestens 50 Gew.-% der Formulierung ausmacht, das die Freisetzung steuernde Mittel in Form einer wäßrigen Suspension oder Emulsion oder einer Wasser und organisches Lösemittel enthaltenden Lösung vorliegt.

2. Pharmazeutische Formulierung nach Anspruch 1, wobei das die Freisetzung steuernde Mittel [i] ein Methacrylsäure/Ethylacrylat-Copolymer oder ein Gemisch des Copolymers mit Ethylcellulose ist.

3. Pharmazeutische Formulierung nach einem der vorangehenden Ansprüche, wobei die physiologisch aktive Substanz [ii] 5-{2-[2(o-Ethoxyphenoxy)ethylamino]propyl}-2-methoxy-benzolsulfonamid-hydrochlorid (YM-12617) ist.

4. Pharmazeutische Formulierung nach einem der vorangehenden Ansprüche, wobei die Produktkörner Durchmesser von 0,1 bis 1,5 mm aufweisen.

5. Körnige Formulierung nach einem der vorangehenden Ansprüche in Form von Tabletten oder Kapseln.

## Revendications

1. Une formulation pharmaceutique à libération contrôlée qui est un produit de granulation,
caractérisée en ce que les granulés ne se désintègrent pas mais libèrent graduellement la substance physiologiquement active dans le tractus gastro-intestinal et qu'ils peuvent être obtenus par un procédé comprenant l'addition [i] d'un agent macromoléculaire de contrôle de libération insoluble dans l'eau, choisi parmi les polymères d'acide acrylique et les copolymères d'acide acrylique, à un mélange de [ii] une substance physiologiquement active et [iii] de cellulose cristalline en tant que substance porteuse et la granulation du mélange résultant, ladite cellulose cristalline constituant au moins 50 % du poids de la formulation, l'agent de contrôle de libération étant sous la forme d'une suspension ou d'une émulsion aqueuse ou d'une solution aqueuse dans un solvant organique.

2. Une formulation pharmaceutique telle que revendiquée à la revendication 1,
dans laquelle l'agent de contrôle de libération [i] est un copolymère d'acide méthacrylique acrylate d'éthyle ou un mélange dudit copolymère et d'éthylcellulose.

3. Une formulation pharmaceutique telle que revendiquée dans une quelconque revendication précédente,
dans laquelle la substance physiologiquement active [ii] est du chlorhydrure de 5-{2-[2(o-éthoxyphénoxy)éthylamino]propyl}-2-méthoxy-benzènesulfonamide.

4. Une formulation pharmaceutique telle que revendiquée dans une quelconque revendication précédente,
dans laquelle les granulés de produit ont des diamètres de 0,1 à 1,5 mm.

5. Une formulation pharmaceutique selon une quelconque revendication précédente, sous forme de comprimés ou de capsules.
